# EUROPEAN PATENT APPLICATION

(11) **EP 4 338 759 A1**
(43) Date of publication of application: **20.03.2024**
(21) Application number: 22195594.1
(22) Date of filing: 14.09.2022
(51) Int. Cl.: A61L 2/10

(54) **A DISINFECTION DEVICE AND METHOD FOR DISINFECTION OF MEDICAL EQUIPMENT**

(71) Applicant: Viobac ApS, 2500 Valby (DK)
(72) Inventor: KØPPEN, Kasper Storm, 2500 Valby (DK); ANDERSEN, Mads Ørbæk, 2500 Valby (DK)
(74) Representative: AWA Denmark A/S

(57) **Abstract**

A disinfection device for disinfection of medical equipment, the disinfection device comprising a housing comprising a disinfection chamber, the disinfection chamber being adapted to at least partly enclose a section of a medical equipment; at least one ultraviolet light emitting diode (UV-LED) configured to emit UV-light directly into the disinfection chamber; a control unit configured to control the UV-LED; and an integrated power source connected to the UV-LED; wherein the control unit is further configured to run a plurality of disinfection cycles each comprising an onperiod of UV-light emission from the UV-LED and an off-period of no UV-light emission from the UV-LED.

## Description

### Technical field

The present invention relates to a disinfection device and method for disinfection of medical equipment. More specifically the invention relates to a device and method for disinfection of medical equipment by use of germicidal UV-light.

### Background

Sterilisation of medical equipment, to minimise the risk of infection of patients during medical treatment, is a well-established procedure in healthcare settings. However, the use of sterile medical equipment is not always enough to ensure patient safety. In treatments where an invasive catheter or tube is applied, infections may be caused by microbes migrating along the catheter or tube, thus continues disinfection or replacement is therefore required. Different types of point-of-care UV-light disinfection devices exist, one example being described in WO2020200389, and while such devices might provide good disinfection, further optimisation is desired when it comes to use time, risk of overheating, and general applicability in humans, minimising the exposure of patients, personal and/or medical equipment of UV-light, which in too high dosages may result in health risk or material degradation.

### Summary

It is an object of the present invention to overcome at least some of these problems, and to provide an improved disinfection device and method for disinfection of medical equipment. It is especially desired to provide a disinfection device and method, which allows maintaining or improving the safety and the quality of the disinfection even over long periods of use.

According to a first aspect of the invention, this and other objects are achieved by providing a disinfection device for disinfection of medical equipment, the disinfection device comprising; a housing having a disinfection chamber, the disinfection chamber being adapted to at least partly enclose a section of a medical equipment; at least one ultraviolet light emitting diode (UV-LED) configured to emit ultraviolet (UV) light directly into the disinfection chamber; a control unit configured to control the UV-LED; and an integrated power source connected to the UV-LED; wherein the control unit is further configured to run a plurality of disinfection cycles each comprising an on-period of UV-light emission from the UV-LED and an off-period of no UV-light emission from the UV-LED.

With this device configuration the usage time of the device is increased compared to the current state of the art, since the disinfection happens in a plurality of disinfection cycles, there will be no or very limited power consumption in the off-periods, and hence the power consumption of the device will be lowered. Furthermore, the off-periods will result in a reduced heat generation, which may contribute to increasing patient safety as the risk of the disinfection device over-heating is reduced. Thirdly, as the UV-light emission only occurs during on-periods the overall total UV-dose emitted will be lowered compared to continues light emission, which improves the safety for patients and personal in the surrounding environment, as well as lower degradation of the medical equipment itself.

The goal of running a plurality of disinfection cycles is to achieve disinfection of at least a section of a medical equipment. By disinfection is meant exposing the section of the medical equipment for a high enough UV-dose over a period of time to achieve at least a 1-log reduction of harmful microbes residing on and moving along the medical equipment. This reduction of harmful microbes should be achieved while limiting the emission of UV-light, so the device will consume less power, have reduced heat generation and the UV exposure to the surrounding environment is limited.

The housing may comprise a through hole extending throughout the housing and adapted to enclose at least a section of an elongate medical equipment, such as a catheter, tube, cable or wire, and the disinfection chamber may form part of the through hole. This allows the disinfection device to be mounted onto an elongate medical equipment and be used for preventing harmful bacteria etc. from travelling along the elongate medical equipment into the body of the patient. Additionally or alternatively, the housing may include a proximal end, a distal end, an attachment portion, and a through hole extending through both the attachment portion and the disinfection chamber, the through hole having a distal opening positioned at the distal end of the housing and leading into the disinfection chamber, the attachment portion being configured to retain a portion of a medical equipment such as a catheter tube in the through hole. A centre axis may extend from the proximal end of the housing to the distal end of the housing. The through hole may extend in parallel to the centre axis. The proximal end of the housing is intended to be oriented towards the patient and the distal end of the housing is intended to be oriented away from the patient. This may ensure that germs moving along the medical equipment towards the patient are eliminated before entering the patient.

The housing may alternatively be configured to enclose the disinfection chamber completely, with no through hole extending throughout the device.

The housing may comprise or consist essentially of a material, such as a polymer material, configured to absorb UV-light at least at the same wavelength of the light emitted by the at least one UV-LED. The housing may comprise a material which reflect the UV-light emitted by the UV-LED. The housing may comprise or consist essentially of a combination of materials which absorb and reflect UV-light, respectively. The use of such materials may help to achieve a disinfection device where the UV-light emitted by the UV-LED is used optimally for disinfection of the medical equipment, while at the same time preventing or reducing UV-light emission from the disinfection device to the surroundings. At present it is particularly envisaged to use material configured to absorb UV-light at the edges of the disinfection chamber, such as a proximal opening of a through hole at the proximal end, i.e. the opening facing the patient during use.

The housing may extend circumferentially around the centre axis. The disinfection chamber and/or the attachment portion of the housing may extend circumferentially around the centre axis.

The diameter of the through hole of the attachment portion may correspond substantially to the outer diameter of the medical equipment which it is designed to hold. The medical equipment may e.g. be a catheter tube or medical guidewire, to be positioned in the through hole. The attachment portion may comprise at least one rib configured to retain a section of the medical equipment being positioned in the through hole. The attachment portion may be configured to axially retain and/or radially retain a section of a medical equipment positioned in the through hole.

The disinfection chamber may have an air-filled cavity, even when a medical equipment is positioned in the through hole, thus ensuring an air gap between the disinfection chamber and medical equipment.

The through hole may have a substantially circular cross-section perpendicular to the centre axis substantially from the distal end of the through hole to the proximal end of the through hole. The through hole may have a varying cross-section perpendicular to the centre axis substantially from the distal end of the through hole to the proximal end of the through hole. The centre axis of the housing may form a centre line of the through hole.

The distal opening may be positioned at an end of the through hole. The distal opening may be positioned adjacent to the disinfection chamber. The distal opening may lead directly to the disinfection chamber. The distal opening may be substantially circular, to provide a substantially uniform radial extent of the air gap around the typically cylindrical catheter tube. The distal opening may be of substantially same shape as the medical equipment, e.g. circular to contain a section of a catheter tube or medical guidewire.

The distal opening may have a sufficiently large diameter in order to prevent germs to cross the air gap from the outer surface of the medical equipment to the housing.

The disinfection device may comprise more than the at least one UV-LED, e.g. at least two, three, four, five, or even more UV-LEDs.

The at least one UV-LED is configured to emit UV-light into the disinfection chamber of the housing to disinfect the outer and possibly even the inner surface of a section of a medical equipment positioned in the disinfection chamber.

The disinfection device may comprise a circuitry connecting the integrated power source with other electronic parts, such as the control unit and UV-LED. The power source, such as a battery, supplies the at least one UV-LED with power.

The control unit may comprise any circuit and/or device suitably adapted to perform the functions of running the plurality of disinfection cycles. The control unit may comprise general purpose or proprietary programmable microprocessors, such as Digital Signal Processors (DSP), Application Specific Integrated Circuits (ASIC), Programmable Logic Arrays (PLA), Field Programmable Gate Arrays (FPGA), a central processing unit (CPU), microcontroller unit (MCU), special-purpose electronic circuits, etc., or a combination thereof. The control unit may be provided with a receiver, a transmitter and/or a transceiver for receiving and transmitting signals either through a wired or a wireless connection. The control unit may comprise a volatile and/or non-volatile memory such as, Random Access Memory (RAM), Read-only memory (ROM), Electrically Erasable Programmable Read-Only-Memory (EEPROM), Flash memory, etc. The control unit may comprise a data storage. The data storage may store one or more instructions to run disinfection cycles of different patterns of on-period to off-period composition. The data storage may store instructions for calibration processes. The data storage may store patient information, such as a specific treatment program of disinfection cycles. Storing instructions to run disinfection cycles of different patterns of on-period to off-period composition may allow the use of one disinfection device for several different purposes, such as targeting different kinds of bacteria. It may also allow the disinfection process to change over time, for example starting with comparatively long on-periods to achieve an initial disinfection then changing to comparatively shorter on-periods for maintaining the disinfection over time. The plurality of disinfection cycles may follow a pattern where on-periods and off-periods are of the same duration in every disinfection cycle of the plurality of disinfection cycles. e.g. 10 seconds on and 10 seconds off.

The plurality of disinfection cycles may follow a pattern where on-periods and off-periods are of different duration in every disinfection cycle of the plurality of disinfection cycles. E.g. 30 seconds on and 300 seconds off, or 30 seconds on and 10 seconds off.

The plurality of disinfection cycles may follow a more complex pattern, where on-periods and off-periods changes duration from disinfection cycle to disinfection cycle in the plurality of disinfection cycles, e.g., disinfection cycle 1; 30 seconds on and 300 seconds off, disinfection cycle 2; 0.5 seconds on and 10 seconds off, disinfection cycle 3; 10 seconds on and 10 seconds off, etc.

In some embodiments at least one disinfection cycle of the plurality of disinfection cycles comprises an on-period with a duration of at least 0.5 second. Experiments has shown a good degree of disinfection when using at least 0.5 second of duration for on-periods. However, as microbes need different UV-doses to be eliminated the exact duration needed depends on the target microbe and desired degree of disinfection. The UV-dose dependents and the intensity (irradiance) from the UV-LED over time, and the efficacy for different microbes also depends on the wavelength of the emitted UV-light. A person skilled in the art may setup a small series of simple experiments, without suffering undue effort, to establish a specific calibration of duration of on-periods to meet a specific desired need, e.g, a required UV-dose for a 3-log reduction of E. Coli has been described as 5.5 mJ/cm², while 7.8 mJ/cm² has been described for S. Aureus, and 16.8 mJ/cm² for Pseudomonas Aeruginosa.

In some embodiments the off-period has a duration of at least twice the duration of the on-period, for each disinfection cycle of the plurality of disinfection cycles. Having an off-period of at least twice the duration of the on-period provide a device with less power consumption and potentially also less UV exposure to the environment. As with choosing the right duration of on-period, the duration of off-period may be subject to calibration and/or testing in order to establish the most optimal duration for a specific microbe or degree of disinfection.

In some embodiments the UV-LED is configured to emit UV-light in the wavelength range of 100-400 nm, preferably 200-315 nm, more preferred 260-300 nm. Thereby, a device which may emit germicidal UV-light is provided. The specific wavelength may be subject to calibration and/or testing in order to establish the most optimal wavelength for a specific microbe or degree of disinfection, power consumption and material degradation of the medical equipment.

In some embodiments a total UV-dose emitted by the UV-LED is at least 20.833 mJ/cm² measured over 30 min, or 41.66 mJ/cm² measured over 1 hour, 500 mJ/cm² over 12 hours or 1000 mJ/cm² measured over 24 hours. Thereby is provided a device that may achieve at least a 1-log reduction of harmful microbes within any one of the abovementioned timeframes. By UV-dose is meant UV intensity/irradiance delivered by the UV-LED times the duration of UV-light exposure;

UV-irradiance x time = UV-dose, where the intensity/irradiance are to be measured by a calibrated optical probe no more than 1 cm away from the UV-LED.

In some embodiments the integrated power source is a primary cell battery. A primary cell battery offers a compact solution, with a high energy density and longer storage lifetime. The primary cell battery may be a zinc-air battery or lithium manganese dioxide battery, or any other suitable primary cell battery.

In some embodiments the disinfection device is a single use device, and the primary cell battery is designed for short-term usage with enough power capacity for 1 day, preferably 3 days, more preferred 7 days of continuous use. Alternatively, the primary cell battery is designed for medium-term usage with enough power capacity for 7 days, preferably 14 days, more preferred 30 days of continuous use. Alternatively, the primary cell battery is designed for long-term usage with enough power capacity for 30 days, preferably 45 days, more preferred 60 days of continuous use. Thereby minimising the need for change of device, limiting the burden of use for patients and personnel.

In some embodiments the device further comprising an antibacterial or bacteria-repellent coating. This will further reduce the risk of infection of a patient by limiting microbial migration and/or by increasing the elimination of microbes.

In some embodiments a UV reflection zone is provided in the disinfection chamber. By reflecting UV-light, it will increase the UV exposure and thereby potentially increase the efficiency of the disinfection.

In some embodiments the disinfection device has a length, a width, and a height, wherein the length is maximum 0.15 m measured along the longest axial length of the device and the width and the height are both maximum 0.1 m measured in a direction perpendicular to the axial length. Consequently, a device which is compact in size and streamlined is provided. A compact size improves the user experience for the patients, as the device will weigh less and take up less space. Additionally, it makes the device ideal as a point-of-care device.

In one embodiment that currently seems advantageous the disinfection de-vice has a length, a width, and a height, wherein the length is maximum 0.05 m measured along the longest axial length of the device and the width and the height are both maximum 0.03 m measured in a direction perpendicular to the axial length

Additionally or alternatively, the disinfection device has a total volume of maximum 0.0015 m³

In one embodiment that currently seems advantageous the disinfection de-vice has a total volume of maximum 0.000045 m³.

In some embodiments the disinfection device has an open configuration and a closed configuration, wherein the disinfection device is in the closed configuration during use.

The open configuration may enable placement of the section of the medical equipment into the disinfection chamber and wherein the closed configuration encloses the UV-LED such that no UV-light is directly emitted outside of the disinfection chamber. In the open configuration, a radial access way may be provided to the through hole, so that a medical equipment can be radially inserted into the through hole. This may allow the disinfection device to be attached to an ex vivo section of a medical equipment with a section already positioned in vivo in a patient, and thus increase the usability of the device.

Additionally or alternatively, the housing comprises, or consist of, two portions interconnected by a hinge, such as a living hinge, a pin-and-knuckle, or a floating hinge, the hinge being settable to the open configuration and the closed configuration, wherein a radial access way to the through hole is provided in the open configuration, so that a section of the medical equipment can be radially inserted into the through hole, and/or wherein the two portions of the housing surround the through hole in the closed configuration, so that the section of the medical equipment already present in the attachment portion of the housing can be at least radially retained.

This may provide the advantage of a particularly mechanically simple arrangement.

The two portions may be positioned at a distance to each other in the open configuration.

The hinge may be a living hinge, which may be a particularly suited for this purpose as it is easier to clean the hinge. The hinge may alternatively be a pin-and-knuckle hinge or a floating hinge.

The hinge may comprise an inner cavity through which a part of device integrated electronics or circuitry may run.

Additionally or alternatively, the two portions of the housing may be in the form of halves of the housing and be movable around a hinge axis of the hinge, the hinge axis being parallel to a centre axis of the through hole.

In some embodiments activation of a switch or sensor connected to the control unit prohibits emission of UV-light when the disinfection device is in the open configuration. An advantage of stopping emission of light in the open configuration may include preventing leakage of UV-light with potentially skin irritating, eye damaging, or even carcinogenic effects as well as saving energy. The switch or sensor may work together with a activation device, wherein the switch or sensor is configured to cause the at least one UV-LED to start emitting UV-light when the sensor detects a signal from the activation device e.g. to indicate that the housing is in the closed configuration. The signal from the activation device may be caused by a magnetic field, a current or a mechanical arrangement. An advantage of starting emission of light in the closed configuration may include improving the disinfection capability of the device as it is not necessary to switch the device on once fitted onto a medical equipment.

Additionally or alternatively, the switch or sensor may be configured to cause the at least one UV-LED to stop emitting UV-light when the detector device detects that the housing is in the open configuration. The switch or sensor may be connected to the control unit, or alternatively, the switch may simply close or interrupt a circuitry of the device to cut or connect to the UV-LED with power from the integrated power source.

According to a second aspect of the invention, the object is achieved by providing a method for disinfection at least a section of a medical equipment, the method comprising; placing a disinfection device in contact with at least a section of the medical equipment, such that the disinfection device at least partly encloses the section of the medical equipment; and prompting a control unit comprised by the disinfection device to run a plurality of disinfection cycles each comprising an on-period of UV-light emission from an UV-LED and an off-period of no UV-light emission, wherein the UV-light emitted during the on-period is emitted directly onto the section of the medical equipment intended to be disinfected. Thereby providing a method of disinfection which provides good disinfection with low power consumption, low heat generation and limited UV-light exposure to the surroundings.

In some embodiments the on-period has a duration of at least 0.5 second. Longer periods of continuous light emission may also increase disinfection rate as microbial DNA and/or RNA repair mechanisms cannot keep up with the damage provided by the continuous UV light emission.

In some embodiments the off-period has a duration of at least twice the duration of the on-period, in each disinfection cycle. Longer off-period between on-periods provides a method which consume less power and have a lower UV exposure to the environment.

Some embodiments further comprising running the plurality of disinfection cycles for at least 1 hour. Thereby enabling a continued UV emission to accumulate into a total UV-does over the period of 1 hour or longer.

Some embodiments further comprising running the plurality of disinfection cycles for up to 60 days. Current investigations show good results for use of the de-vice for continuous disinfection of urinary catheters. These are often used over prolonged periods, thus enabling continuous disinfection will be advantageous to keeping patient free of infections. Similar good results are expected for all kinds of medical catheters and other elongate medical equipment.

Some embodiments further comprising prompting the control unit to stop the plurality of disinfection cycles, removing the disinfection device from the medical equipment, and disposing of the disinfection device. The disinfection device is intended as a single use device to mitigate the risk of transferring infections between users. Proper disposal is hence important.

In some embodiments the UV-LED is turned off, thereby stopping any emission of UV-light, when the disinfection device is in an open configuration. As described with respect to the first aspect, stopping any emission of UV-light, when the disinfection device is in an open configuration works as a safety feature to ensuring minimal UV exposure from the disinfection device to the surrounding.

Additionally or alternatively, the object according to the second aspect of the invention may be achieved by providing a method for disinfection at least a section of a medical equipment, the method comprising; a. at least partly enclosed a section of the medical equipment in a disinfection device; b. exposing for a duration of at least 0.5 second the section of the medical equipment for UV-light emitted by an UV-LED comprised by the disinfection device, c. turning off the emission of UV-light for a duration of at least twice the duration of the previous step b.; d. repeating step b. and c. for a total duration of at least an hour.

Some embodiments further comprise turning off the emission of UV-light if at least one of; the medical equipment is removed from contact with the disinfection device; and the disinfection device is opened.

Embodiments and advantages described with reference to the first aspect also applies to the second aspect and vice versa unless otherwise stated.

### Brief description of figures

Fig. 1 shows a perspective view of a disinfection device attached to a section of a catheter tube,
Fig. 2a shows a side view of the disinfection device and catheter tube of Fig. 1,
Fig. 2b shows a side cross-sectional view of the disinfection device and catheter tube along cross-sectional line B-B of Fig. 2a,
Fig. 3 shows a perspective view of a disinfection device in an open configuration without a catheter tube,
Fig. 4a shows a side view of the disinfection device of Fig. 3,
Fig. 4b shows a cross-sectional view of the disinfection device along cross-sectional line C-C of Fig. 4a,
Fig. 4c shows a cross-sectional view of the disinfection device along cross-sectional line D-D of Fig. 4a,
Fig. 5 corresponds to Fig. 1 but showing a different embodiment of the disinfection device,
Fig. 6 corresponds to Fig. 3 but showing the embodiment in Fig. 5, and
Fig. 7 shows the disinfection device in Figs 5 and 6 when in use on a urinary catheter, and
Fig 8a-e shows different embodiments of disinfection cycles.

### Detailed description

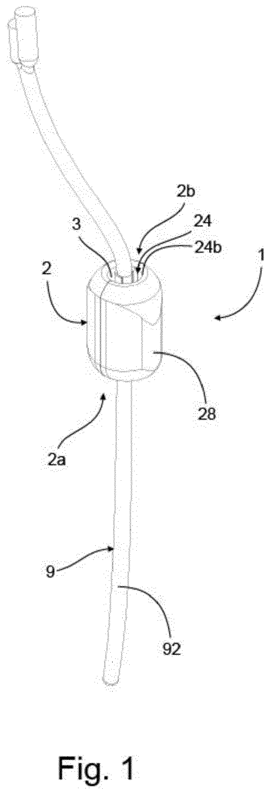

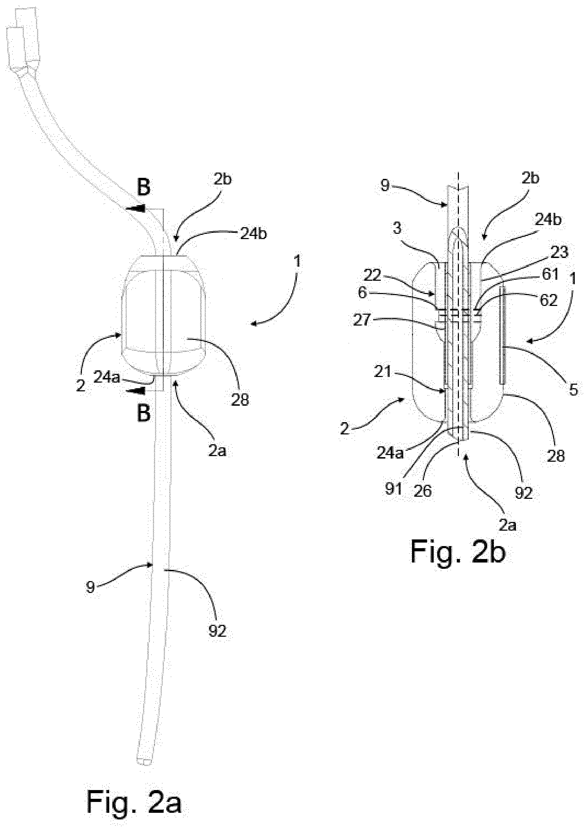

Referring first to Fig. 1 and 2a, a disinfection device 1 is shown with a medical equipment 9 in the form of a catheter tube 9 extending there through. While reference will be made primarily to a catheter tube 9 it is to be understood that this is only an example and that the disinfection device may also be used on a wire or other elongate item projecting into the body of a patient.

The disinfection device 1 com-prises a housing 2 with a proximal end 2a, a distal end 2b, and an outer sur-face 28. The proximal end 2a of the housing 2 is intended to be oriented towards a patient in a use state of the disinfection device and the distal end 2b of the housing 2 is intended to be oriented away from the patient. The outer surface 28 of the housing 2 is facing the exterior of the disinfection device 1 and has a streamlined, curved shape from the proximal end 2a to the distal end 2b without sharp edges or corners in order to prevent causing a pressure ulcer if the disinfection device 1 should end up beneath the patient. The housing 2 comprises a through hole 24 with a circular proximal opening 24a located at the proximal end 2a of the housing 2 and a circular distal opening 24b located at the distal end 2b of the housing 2.

Turning to Fig. 2b, a centre axis 26 extends from the proximal end 2a of the housing 2 to the distal end 2b of the housing 2. The centre axis 26 of the housing 2 forms a centre line of the through hole 24. The through hole 24 has a circular cross-section perpendicular to the centre axis 26 all throughout from the distal opening 24b at the distal end 2b of the housing 2 to the proximal opening 24a at the proximal end 2a of the through hole 24.

The housing 2 comprises an attachment portion 21, a disinfection chamber 22, and the through hole 24 extending through both the attachment portion 21 and the disinfection chamber 22. From the distal opening 24b the through hole 24 leads directly into the disinfection chamber 22. A section of a catheter tube 9 is retained in the through hole 24 by the attachment portion and a centring member 27.

The disinfection chamber 22 comprises a wall surface 23 extending between an UV-LED 6 and the distal opening 24b. The wall surface 23 forms an interior circumferentially and axially extending surface of the through hole 24. The distal opening 24b forms a border of the wall surface 23.

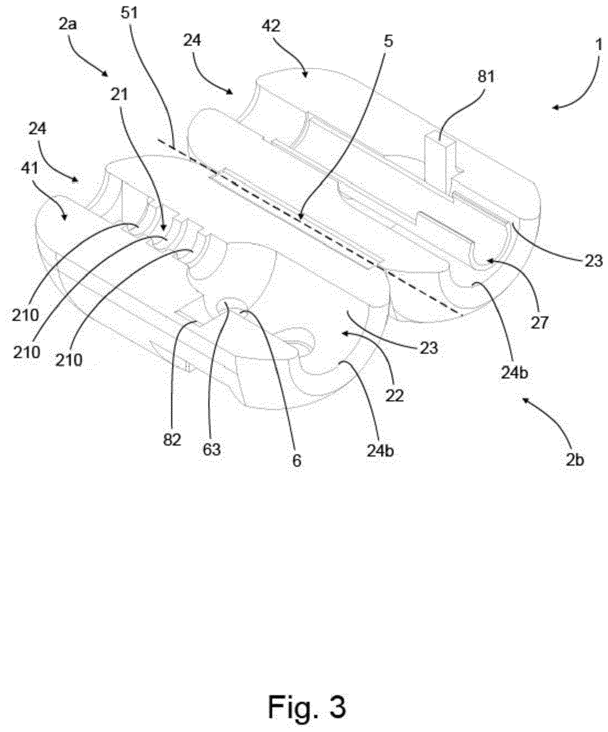

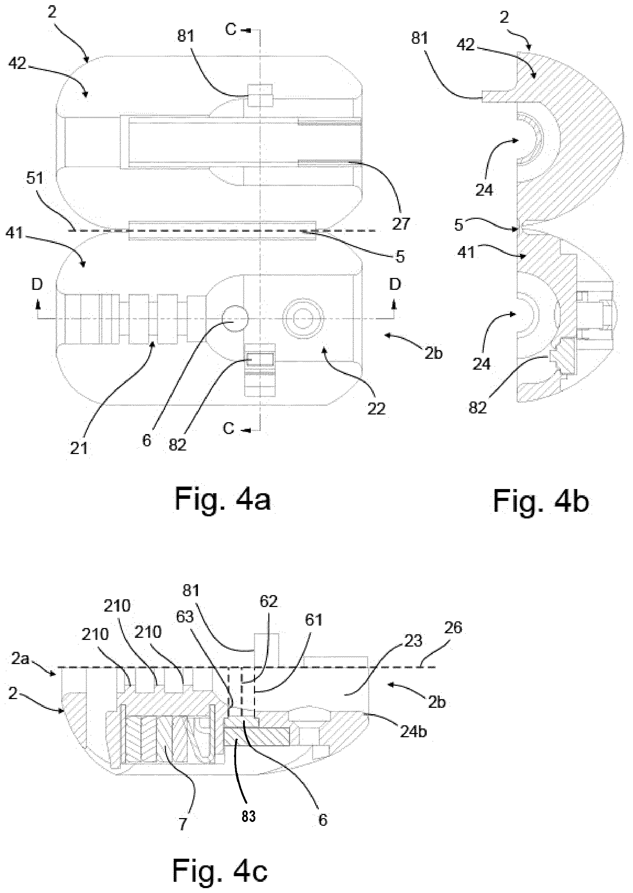

Referring now to Fig. 3 which shows the disinfection device 1 in an open configuration. The housing 2 comprises a floating hinge 5 interconnecting a first half 41 and a second half 42 of the housing 2. The halves 41, 42 of the housing 2 are movable around a hinge axis 51 of the hinge 5, which is parallel to the centre axis 26. The hinge 5 allows the disinfection device 1 to be set in the open configuration as shown in Figs. 3 and 4a-4c, and in an open configuration as shown in Figs. 1 and 2a-2b. The open configuration provides radial access to the through hole 24 so that a section of a catheter tube, or other elongate medical equipment can be radially inserted into the through hole 24 and attached to the housing 2 by the attachment portion 21 of the housing 2. In the open configuration, the two halves 41, 42 radially surround the catheter tube 9 in the through hole 24 so as to radially retain the catheter tube 9 in the through hole 24.

In this embodiment the disinfection device 1 comprises a centring member 27 in the form of a tubing holder as seen in Fig. 3. In the closed position the centring member 27 attaches to the catheter tube 9 by elastically deforming and snapping onto the outer surface 92 of the catheter tube 9. The centring member 27 biases the catheter tube 9 towards the centre line, i.e. the centre axis 26, of the disinfection chamber 22 as best seen in Fig. 2b. The centring member 27 consist essentially of an ultraviolet translucent or ultraviolet transparent material.

The attachment portion 21 of this embodiment comprises three ribs 210 configured to retain a section of the catheter tube 9 being positioned in the through hole 24. The diameter of the through hole 24 is greater in the disinfection chamber portion of the housing than a diameter of the through hole 24 at the ribs 210 of the attachment portion 21 of the housing 2, so that when the catheter tube 9 is retained in the through hole 24 by the attachment portion 21 of the housing 2, an air gap 3 is formed between the disinfection chamber 22 and the catheter tube 9, as can be seen between the centring member 27 and the wall surface 23 of the disinfection chamber 22 in Fig. 2b.

The UV-LED 6 is in the form of a light emitting diode configured to emit germicidal UV-light with a wavelength in the range of 100-400 nm, preferably 200-315 nm, and more preferred 260-300 nm into the disinfection chamber 22 of the housing 2. The UV-LED 6 is positioned in and emits light into the disinfection chamber 22 to disinfect the portion of the catheter tube 9 residing the disinfection chamber.

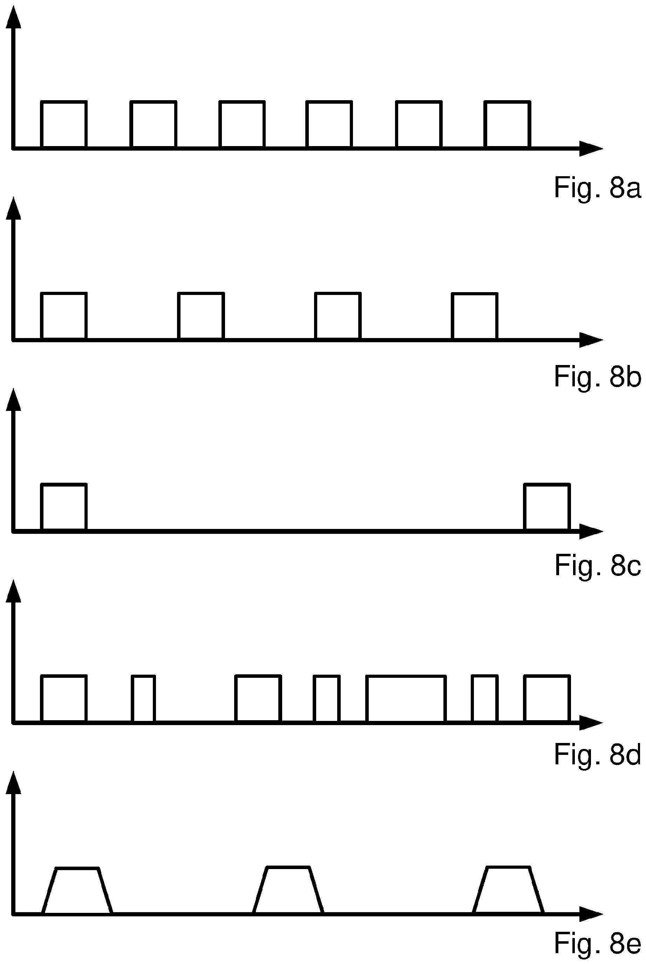

A control unit 83 is configured for controlling the UV-LED 6 to emit the UV-light in a number of disinfections cycles comprising of on-periods of light emission and off-periods of no light emission. The disinfection cycles will be described in more detail with reference to Fig.8a-e.

The wall surface 23 of the disinfection chamber 22 consist essentially of a polymer material configured to at least partially absorb UV-light.

The UV-LED 6 is positioned in a light well 63 as best seen in Figs. 3 and 4c which ensures that light emitted from the UV-LED 6 exits the light well 63 substantially as a light cylinder 61 centred around an optical axis 62 of the UV-LED 6 as shown by broken lines in Fig. 4c. The optical axis 62 of the UV-LED 6 is substantially perpendicular to the centre axis as seen on Fig. 4c, so that the light rays directly emitted by the UV-LED 6 is received and at least partially absorbed by the oppo-site side of the wall surface of the disinfection chamber 22, the centring member 27, or the outer surface 92 of the catheter tube 9. This has the advantage that the intensity/irradiance of the UV-light reaching the distal opening 24b of the through hole 24 is kept at a minimum.

As best seen in Figs. 4a - 4c, the disinfection device 1 further comprises a activation device 81 in the form of a protruding pin, a switch 82, a control unit 83, and a power source 7, in the form of a primary cell battery. A circuitry interconnects the control unit 83, the switch 82, the UV-LED 6, and the power source 7. The power source is configured for supplying power to the circuitry and thereby the control unit 83 and UV-LED 6. The activation device 81 is positioned on the second half 42 of the housing 2 and the switch 82 is positioned on the first half 41 of the housing 2.

In the open configuration, as shown in Fig. 3, the activation device 81 is positioned at a distance to the switch 82 which disconnects the power source 7 from the UV-LED 6 causing the UV-LED 6 to stop emitting the UV-light .

In the closed configuration, as shown in Figs. 1 and 2a-2b, the activation device 81 contacts the switch 82 connecting the power source 7 to the UV-LED 6, causing the UV-LED 6 to start emitting UV-light in pre-programmed disinfection cycles.

Alternatively, instead of disconnecting the power source 7 from the UV-LED 6, the switch 82 may prompt the control unit 83 to turn on and off the UV-LED 6. In that case the device 1 will preferably have two or more switches 82, of either the same or multiple types, thereby securing a redundancy and safety mechanism of turning off the emission of UV-light from the UV-LED 6 when the device 1 is in the open configuration, thus making the device 1 single fault safe. Examples of suitable switches for such an application are Hall effect sensors and reed switches.

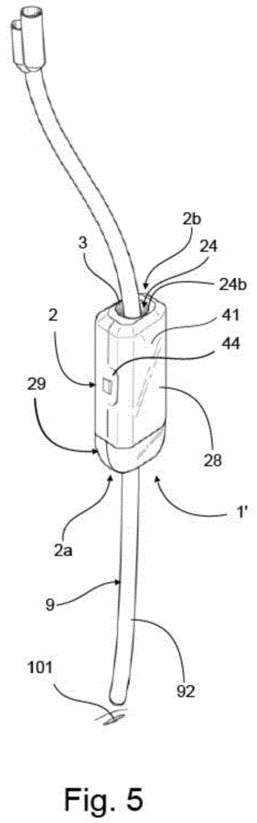

A different embodiment of a disinfection device 1' is shown in Figs 5-7. In these figures and in the description relating thereto, the same reference numbers are used as in Figs 1-4 for features having the same function even though they are not necessarily identical. In order to avoid undue repetition only the differences, between the embodiment of Fig 5-7 and the one described with reference to Fig. 1-4, will be described in detail.

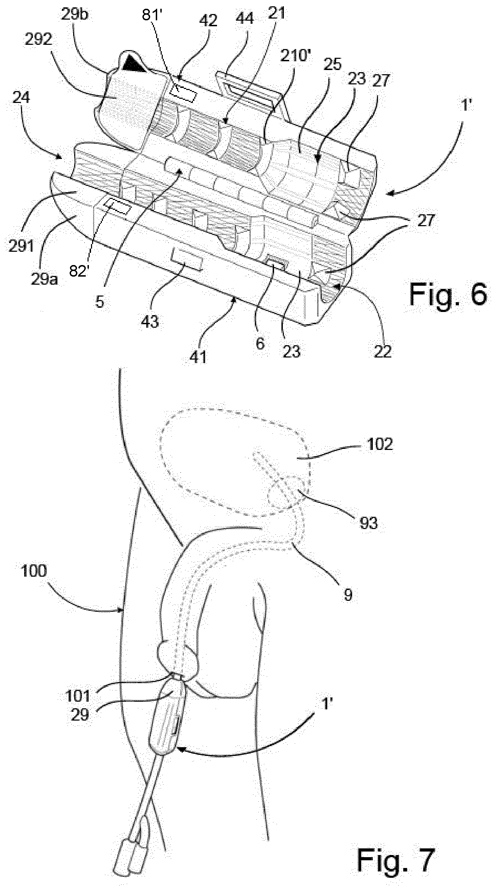

Fig. 5 shows a disinfection device 1' mounted on a catheter tube 9 and in Fig. 6 the disinfection device is shown in an opened configuration. As may be seen, this disinfection device 1' is of a less rounded outer shape than the disinfection device 1 in Figs 1-4, and the cross-section of the through hole 24 throughout the device 1 is not completely circular.

In order to be able to attach the disinfection device 1' on the catheter tube 9 as close to the opening 101 in the patient's body 100 as possible (see Fig. 7) and thereby minimize the area on the catheter tube via which bacteria can by-pass the device, a soft, resilient foam/silicone material in the form of a proximal part 29 has been added at the proximal end 2a of the device 1'. When a catheter, or another elongate medical equipment, is inserted through an opening 101 in the body 100 of a patient, the proximal part 29 is positioned close to the opening 101 in the body 100 of the patient as shown in Fig. 5 and 7. In Fig. 7 the catheter tube 9 is a urinary catheter inserted in the urethra of a male patient, but it is might as well be used for a female patient or for a different type of elongate medical equipment, such as an abdominal catheter or the wire of an temporary pacemaker.

The provision of the proximal part 29 reduces the risk of the disinfection device 1' inflicting a pressure on the patient's body, which may be particularly unpleasant when used at the genitalia as shown in Fig. 7 and which may also result in skin damages and hence an increased risk of infection.

Another advantage of the soft and resilient proximal part 29 is that the risk of the catheter tube 9 being pulled or pushed on when the patient moves is reduced. In the example in Fig. 7 the catheter tube 9 is being held in place in the urinary bladder 102 of the patient by means of a balloon 93 arranged on the section of the catheter tube extending into urinary bladder. The balloon prevents the catheter tube 9 from being pulled out, but does not prevent it from being pushed further in. The provision of the proximal part 29 allows the disinfection device 1' to be positioned very close to the opening in the patient's body, in this case down to about 1 mm from the opening, and this in itself reduces the risk of the catheter tube 9 being pushed inwards. In addition, the resilience of the material of the proximal part 29 means that it may compensate for some movements.

As described above with reference to Fig. 3 the disinfection device 1' in Figs 5-7 also consists of two halves 41, 42, which are interconnected at a hinge 5 as may be seen in Fig. 6. The proximal part 29 is also consisting of two halves 29a, 29b each associated with a respective half 41, 42 of the de-vice.

To ensure that the disinfection device 1' closes tightly around the catheter tube 9, a male lock part 43 is provided in the first half 41 and a female lock part 44 is provided on the second half 42. When moving the disinfection device from the open configuration in Fig. 6 to the open configuration in Fig. 5 these two lock parts will snap-lock to each other. In addition, one half 29b of proximal part 29 is provided with an adhesive (not visible), which is covered by a cover sheet 292. Before closing the disinfection device 1' around the catheter tube 9, the cover sheet is to be removed so that the two halves 29a, 29b of the proximal part 29 adhere to each other in the open configuration.

Other means for locking the two halves 41, 42 to each other and/or for connecting the two halves 29a, 29b of the proximal part are also possible, including the use of tape and hook-and-loop type fasteners, such as Velcro. As may be seen in Fig. 6 this embodiment of the disinfection device 1' comprises a series of centring members 27 projecting from the inner wall sur-face 23 at the distal end. Each of these centring members 27 contact the catheter tube 9 and contributes to keeping it centred with the disinfection device. An advantage of using these centring members is that the contact area with the catheter tube is small.

The embodiment in Fig. 5-7 comprises a UV reflection zone 25 on the inner wall surface 23, said zone extending from the rib marked 210' of the attachment portion 21 to the centring members 27. The surface of the UV reflection zone is made from a material, which reflects UV radiation. Due to the reflection of the UV light emitted by the UV-LED 6, the catheter tube 9 extending through the disinfection chamber 22 will be subjected to an in-creased UV exposure compared to if using a corresponding disinfection de-vice without a UV reflection zone or with a wall sur-face configured to absorb ultraviolet light as described with reference to Fig. 3 and 4.

The embodiment in Figs 5-7 also comprises a control unit 83 configured for controlling the UV-LED 6 to emit the UV-light in a number of disinfections cycles comprising of on-periods of light emission and off-periods of no light emission as described with reference to Fig. 4c. The device 1' further comprises a activation de-vice 81', and a switch unit 82', configured to cause the UV-LED 6 to stop emitting UV-light when the disinfection device 1' is opened as described with reference to Fig. 4a-c. The switch unit 82' shown with reference to Fig. 6 comprises two switches, which both work in relation the activation device, thus if one of the switches should fail the device 1' will still have an active safety mechanism of turning off the emission of UV-light from the UV-LED 6 when the device 1' is in the open configuration. In the shown embodiment the switches are a Reed switch and a Hall effect sensor and the activation device 81' is a magnet. However, other choices of type of activation device and switches are equally conceivable within the scope of the invention.

Even though the activation device 81' and switch unit 82' described in respect to this embodiment is placed separately from the male and female lock parts 43, 44 it is a conceivable design of the device 1' having the activation device 81' and switch unit 82' integrated within the male and female lock parts 43, 44. And likewise, both the activation device 81' and switch unit 82' may be integrated in either of the first half 41 and second half 42 of the device 1'.

Lastly turning to Fig. 8a-e, which show some examples of disinfections cycles, which may be programmed into and run by the control unit 83.

The goal of running a plurality of disinfection cycles is to expose a section of a medical equipment, such as the aforementioned catheter 9, to a high enough UV-dose over a period of time to eliminate most of the harmful microbes residing on and moving along it, while limiting the amount of emitted UV-light. By limiting the emission of UV-light, the device 1 will consume less power, generate less heat and the UV exposure caused to the surrounding environment is limited.

A disinfection cycle consists of an on-period where light is emitted from the UV-LED 6 and an off-period where no light is emitted from the UV-LED 6. On each of Fig. 8a-e the horizontal axis illustrates time and the vertical axis illustrates light intensity. A plurality of disinfection cycles is shown in each of Fig. 8a-e. As light is emitted the graph displays a certain light intensity. During each off-period the light intensity is zero. Over a certain period of time the individual light intensities from the on-periods will accumulate and reach a desired UV-dose, defined by the area below the graphs. There are several ways to obtain the desired UV-dose. Fig. 8a illustrates a case were on- and off-periods are equal in duration. In Fig. 8b the duration of the off-periods is twice that of the on-periods. Fig. 8c shows a case were the duration of the off-periods is many times that of the on-periods, here approximately ten times that of the on-period. Fig. 8a-c are illustrative of some likely settings of the plurality of disinfection cycles, however other rrelationship between on- and off-period durations are equally conceivable, including relationships were the off-period is shorter in duration than the on-period, in each disinfection cycle. It is also important to note that consecutive disinfections cycles do not have to be identical, rather the plurality of disinfections cycles may be complex as is illustrated in Fig. 8d. On- and off-periods may vary in duration, both from disinfections cycle to disinfections cycle, and within each disinfections cycle. Lastly, as is illustrated in Fig. 8e, the maximum intensity reached in each on-period may be built up, and phased out, over a fraction of the duration of the on-period and it is also possible to increase and decrease light intensity gradually, which would result in a curvy graph. Likewise, built up and phase out sequences may be of different duration, resulting in non-identical on-periods for the plurality of disinfection cycles. The plurality of disinfections cycles may comprise both the type of disinfection cycles where the intensity goes from 0 to 100% and the type of disinfection cycles were the intensity is built up, arranged in any combination so desired, including on-periods of lower intensity than others.

To achieve a high enough disinfection quality, different configuration of the device may be applied. A person skilled in the art understands that there are numerous ways to configure the device and design the disinfections cycles in order to achieve this objective. Considering wavelength and intensity of the UV-light emitted by the UV-LED, as well as on- and off-periods of the disinfections cycles some examples of full device configurations are provided in the following.
Example 1: 275-280 nm, 30 sec on, 300 sec off, 1 mJ/s/cm².
Example 2: 275-280 nm, 3 sec on, 30 sec off, 1 mJ/s/cm².
Example 3: 275-280 nm, 1 sec on, 5 sec off, 2 mJ/s/cm².
Example 4: 275-280 nm, 60 sec on, 600 sec off, 0.5 mJ/s/cm².
The following is a list of reference numerals used throughout this specification. In case of any doubt, the reference numerals of the following list apply.
- 1: disinfection device
- 2: housing
- 2a: proximal end
- 2b: distal end
- 21: attachment portion
- 210: rib
- 22: disinfection chamber
- 23: wall surface
- 24: through hole
- 24a: proximal opening
- 24b: distal opening
- 25: reflection zone
- 26: centre axis
- 27: centring member
- 28: outer surface
- 29: proximal part
- 29a: first half of proximal part
- 29b: second half of proximal part
- 292: cover sheet on proximal part
- 3: air gap
- 41: first half
- 42: second half
- 43: lock part on first half
- 44: lock part on second half
- 5: hinge
- 51: hinge axis
- 6: UV-LED
- 61: light cylinder
- 62: optical axis
- 63: light well
- 7: power source
- 81: activation device
- 82: switch
- 82': switch unit
- 83: control unit
- 9: catheter tube
- 92: outer surface
- 93: balloon
- 100: body of patient
- 101: opening in patient
- 102: urinary bladder

## Claims

1. A disinfection device for disinfection of medical equipment, the disinfection device comprising:
a housing comprising a disinfection chamber, the disinfection chamber being adapted to at least partly enclose a section of a medical equipment;
at least one ultraviolet light emitting diode (UV-LED) configured to emit UV-light directly into the disinfection chamber;
a control unit configured to control the UV-LED; and
an integrated power source connected to the UV-LED; wherein
the control unit is further configured to run a plurality of disinfection cycles each comprising an on-period of UV-light emission from the UV-LED and an off-period of no UV-light emission from the UV-LED.

2. The disinfection device according to claim 1, wherein at least one disinfection cycle of the plurality of disinfection cycles comprises an on-period with a duration of at least 0.5 second.

3. The disinfection device according to claim 1 or 2, wherein the off-period has a duration of at least twice the duration of the on-period for each disinfection cycle of the plurality of disinfection cycles.

4. The disinfection device according any one of the claims 1 to 3, wherein the UV-LED is configured to emit germicidal UV-light in the wavelength range of 100-400 nm, preferably 200-315 nm, more preferred 260-300 nm.

5. The disinfection device according to any one of the claims 1 to 4, wherein a total UV-dose emitted by the UV-LED is at least 20.833 mJ/cm² measured over 30 min, or 41.66 mJ/cm² measured over 1 hour, or 1000 mJ/cm² measured over 24 hours.

6. The disinfection device according to any one of the preceding claims, wherein the housing comprises a through hole extending throughout the housing and adapted to enclose at least a section of an elongate medical equipment, such as a catheter, tube, cable or wire, and wherein the disinfection chamber forms part of the through hole.

7. The disinfection device according to any one of the preceding claims, wherein the integrated power source is a primary cell battery.

8. The disinfection device according to claim 7, wherein the disinfection de-vice is a single use device and the primary cell battery has enough power capacity for at least 1 day, or 3 days, or 7 days, or 14 days, or 30 days, or 45 days, or 60 days of continuous use.

9. The disinfection device according to any one of the preceding claims, wherein the disinfection device has a total volume of maximum 0.0015 m³.

10. The disinfection device according to any one of the preceding claims, wherein the disinfection device has an open configuration and a closed configuration, wherein the disinfection device is in the closed configuration during use.

11. A method for disinfection at least a section of a medical equipment, the method comprising:
placing a disinfection device in contact with at least a section of the medical equipment, such that the disinfection device at least partly encloses the section of the medical equipment; and
prompting a control unit comprised in the disinfection device to run a plurality of disinfection cycles each comprising an on-period of UV-light emission from an UV-LED and an off-period of no UV-light emission, wherein the UV-light emitted during the on-period is emitted directly onto the section of the medical equipment intended to be disinfected.

12. The method according to claim 11, wherein the on-period has a duration of at least 0.5 second.

13. The method according to claim 11 or12, wherein the off-period has a duration of at least twice the duration of the on-period, in each disinfection cycle.

14. The method according to any one of claim 11 to 13, further comprising running the plurality of disinfection cycles for at least 1 hour.

15. The method according to any one of claim 11 to 14, further comprising running the plurality of disinfection cycles for up to 60 days.
